# EUROPEAN PATENT APPLICATION

(11) **EP 2 270 143 A1**
(43) Date of publication of application: **05.01.2011**
(21) Application number: 09726022.8
(22) Date of filing: 24.03.2009
(51) Int. Cl.: C12N 15/09, C12M 1/00

(54) **METHOD OF GENE TRANSFER INTO CELLS USING ELECTROSPRAY AND APPARATUS THEREFOR**

(30) Priority: 25.03.2008 JP 2008077014
(71) Applicant: Mitsubishi Gas Chemical Company, Inc., Chiyoda-ku Tokyo 100-8324 (JP)
(72) Inventor: IKEMOTO, Kazuto, Niigata-shi Niigata 950-3112 (JP)
(74) Representative: Harrison, Robert John
(86) International application number: PCT/JP2009/055783
(87) International publication number: WO 2009/119559

(57) **Abstract**

The present invention provides a method whereby, in transferring a gene into a cell by contacting the cell with a gene to be transferred into the cell in a container and then electrospraying a spray liquid free from the gene on the cell and gene in the container, the gene can be rapidly and conveniently transferred into the cell with high transfer efficiency while minimizing the degradation of the gene, and an apparatus therefore. A nozzle for electrospraying comprising a tube portion for applying a high voltage, which is made of an electrically conductive substance and located on the side of the spray liquid suction port, and another tube portion for spraying, which is made of an insulating substance and located on the side of the spray liquid ejection port. By using this nozzle, electrospraying can be performed while preventing a discharge phenomenon that causes degradation of the gene. Thus, transfer efficiency of the gene into the cell can be remarkably improved, and the gene can be rapidly and conveniently transferred into the cell.

## Description

### TECHNICAL FIELD

The present invention relates to a method for transferring a gene into a cell by contacting a cell with a gene to be transferred into the cell in a container, and then electrospraying the cell and gene with a spray liquid free from the gene, in which the gene is transferred efficiently into the cell by electrospraying while minimizing the degradation of the gene, and also relates to an apparatus therefor. An electrospraying method which can efficiently transfer an intact gene comprising nucleobases such as DNA and RNA into a cell, and an apparatus therefor are technical means that are very useful in the field of research and application related to medical care, agriculture and the like.

### BACKGROUND ART

Electrospray phenomenon is used as a means for spraying highly-voltaged and charged minute droplets of a spray liquid at high speed toward a counter electrode by applying a high voltage to a spray nozzle so as to collect electric charges at the tip of the nozzle, and passing the liquid through the nozzle tip at which electric charges have accumulated.

As one of the gene transfer methods utilizing the electrospray, mention may be made of particle method. This is a method in which a high voltage is applied to a suspension containing particles so as to spray them on cells when they are passed through a capillary tip (for example, refer to Patent Document 1). In addition, there have been developed a method and an apparatus which continuously and conveniently transfer multiple types of genes into cells by contacting a cell with a gene to be transferred into the cell, and then electrospraying the cell and gene with a liquid free from the gene (for example, refer to Patent Documents 2 and 3).

Therefore, the method for transferring a gene into cells utilizing the electrospray is excellent in that a gene can be conveniently transferred into cells by physical means for applying a high voltage to a spray liquid so as to spray it, but is not always sufficient in transfer efficiency of genes into cells, and is still problematic in that further improvement is required.

During studies on the electrospray which can transfer genes into cells with high transfer efficiency, the present inventor has found that genes comprising nucleobases such as DNA and RNA are degraded with high percentage at the stage of electrospraying treatment, and also this degradation phenomenon happens not only when a spray liquid containing a gene to be transferred into cells is electrosprayed on cells, but also when a spray liquid free from genes is electrosprayed after cells are brought into contact with the gene to be transferred into cells in the container. Such degradation of genes not only lowers transfer efficiency of the substance into cells, but also may lead to lower expression efficiency of the gene or expression of wrong gene information, and thus is extremely inconvenient.

Examples of reports referring to gene degradation by electrospray include a report which shows that fragmentation of DNA occurred when a liquid containing DNA was electrosprayed (for example, refer to non-Patent Document 3), and also a report which shows that not only decrease in molecular weight such as fragmentation, but also increase in molecular weight occurred (for example, refer to non-Patent Document 4). However, these reports only show that the gene degradation occurred at the time of mass analysis, but provide no investigation of cause or countermeasure.

By the way, as an ordinary idea, decrease of voltage to be applied is thought to be effective in order to inhibit generation of fragmentation, but when a gene is transferred into cells, the decrease of voltage to be applied may concurrently lead to decrease of transfer efficiency of the gene, and thus cannot be said to be advisable. In addition, this cannot be said to be an appropriate measure since a higher voltage is often required for the electrospray for transfer of the gene into cells compared with the voltage to be applied at the time of mass analysis. Thus, it has been strongly demanded to provide a method which can transfer a gene sample from multiple specimens into cells with high percentage conveniently whilst the degradation phenomenon of a gene is avoided when the gene is transferred into cells by electrospraying, and an apparatus for conducting the method.

Patent Document 1: United States Patent No. 6746869
Patent Document 2: Japanese Patent Application No. 2006-136851
Patent Document 3: Japanese Patent Application No. 2006-136856
Non-Patent Document 1: J. B. Fenn, M. Mann, C. K. Meng, S. F. Wong, C. M. Whitehouse, Science, 1989, vol. 246, p64-71.
Non-Patent Document 2: Z. Takats, J. M. Wiseman, B. Gologan, R. G. Cooks, Science, 2004, vol. 306, p471-473.
Non-Patent Document 3: E. Nordhoff, F. Kirperkar, P. Roepstorff, Mass Spectrometry Reviews, 1996, 15, P67-138.
Non-Patent Document 4: Xueheng Cheng" David G. Camp, Qinyuan Wu" Ray Bakhtiar, David L. Springer, Brendt J. Morris" James E. Bruce, Gordon A. Anderson, Charles G. Edmonds, Richard D. Smith, Nucleic Acids Research, 1996, Vol. 24, No. 112183-2189.

### DISCLOSURE OF THE INVENTION

### PROBLEMS TO BE SOLVED BY THE INVENTION

The object of the present invention is to provide a method for transferring a gene into cells using an electrospray which can rapidly and conveniently transfer a gene comprising nucleobases such as DNA and RNA into cells with high transfer efficiency while minimizing the degradation of the gene, and an apparatus used in the method.

### MEANS FOR SOLVING THE PROBLEM

The present inventor has intensively studied the cause of gene degradation which occurs during electrospraying and a method for preventing it. As a result, he has found that gene degradation is caused by discharge current; degradation of a gene to be transferred into cells can be inhibited by controlling the amount of electric current flowing through the gene and the cells to not more than 50 µA during electrospraying; and transfer efficiency of the gene into cells is improved dramatically by employing, as an achieving means, an electrospraying nozzle structure which is separated into a tube portion made of an electrically conductive substance for applying a high voltage and a tube portion made of an insulating substance for ejecting a spray liquid so that a step for applying a high voltage to the spray liquid can be conducted separately from a step of ejecting the spray liquid as misty and minute droplets, because this makes it possible to apply the principle of electrospray whilst the electric discharge which occurs at the tube tip and the like can be avoided. Thus, the present invention has been completed.

That is, as described in the following items (1) to (12), the present invention relates to a method which can inhibit gene degradation occurring during electrospraying, and can rapidly and conveniently transfer the gene into cells with high transfer efficiency, and an apparatus used in the method.
(1) A method for transferring a gene into a cell by electrospraying, which comprises bringing a gene to be transferred into a cell in contact with the cell in a container, and then electrospraying the cell and gene in the container with a spray liquid free from the gene, in which, as a nozzle for electrospraying, a nozzle is used which comprises:
   a tube portion for applying a high voltage, which is made of an electrically conductive substance and located on the side of the spray liquid suction port, and
   a tube portion for spraying, which is made of an insulating substance and located on the side of the spray liquid ejection port.
(2) The method for transferring a gene into a cell by electrospraying, according to item (1), wherein, as the nozzle for electrospraying, a nozzle is used in which the shortest distance from the tube portion for applying a high voltage, which is made of an electrically conductive substance and located on the side of the spray liquid suction port, to the tip of the ejection port of the tube portion for spraying, which is made of an insulating substance and located on the side of the spray liquid ejection port, is 5 mm to 500 mm.
(3) The method for transferring a gene into a cell by electrospraying, according to item (1), wherein electrospraying is conducted whilst an amount of electric current flowing through the cell and gene in the container is controlled not to exceed 50 µA.
(4) The method for transferring a gene into a cell by electrospraying, according to item (3), wherein an electrode through which electric current is bypassed is provided between the tip of the ejection port of the tube portion for spraying, which is made of an insulating substance and located on the side of the spray liquid ejection port, and the cell and gene placed in the container, and electrospraying is conducted whilst the amount of electric current flowing through the cell and gene is controlled not to exceed 50 µA.
(5) The method for transferring a gene into a cell by electrospraying, according to item (1), wherein a structure for dropping a spray liquid so as to cut off electrical conduction of the spray liquid is provided between the spray liquid suction port side of the nozzle for electrospraying and a spray liquid transfer line arranged upstream of the suction port, whereby electrospraying is conducted with electric leak being prevented.
(6) An apparatus which is used for conducting the method for transferring a gene into a cell by electrospraying according to any one of items (1) to (5).
(7) A nozzle for electrospraying, which comprises:
   a tube portion for applying a high voltage, which is made of an electrically conductive substance and located on the side of the spray liquid suction port, and
   a tube portion for spraying, which is made of an insulating substance and located on the side of the spray liquid ejection port.
(8) The nozzle for electrospraying, according to item (7), wherein the shortest distance from the tube portion for applying a high voltage to the tip of the ejection port of the tube portion for spraying is 5 mm to 500 mm.
(9) An apparatus for transferring a gene into a cell, which comprises at least a container in which a cell and a gene to be transferred into the cell are placed, a nozzle for electrospraying the cell and gene in the container with a spray liquid free from the gene, and a high voltage power source which applies a high voltage to the nozzle, wherein the nozzle is a nozzle for electrospraying according to items (7) and (8).
(10) The apparatus according to item (9), which further comprises a means which controls an amount of electric current flowing through the cell and gene in the container not to exceed 50 µA.
(11) The apparatus according to item (10), wherein the means which controls the amount of electric current not to exceed 50 µA comprises an electrode through which electric current is bypassed and which is provided between the tip of the ejection port of the tube portion for spraying and the container.
(12) The apparatus according to any one of items (9) to (11), which further comprises a structure for dropping a spray liquid so as to cut off electrical conduction of the spray liquid as an electric leak preventing means between the spray liquid suction port side of the nozzle and a spray liquid transfer line arranged upstream of the suction port.

### EFFECT OF THE INVENTION

By using the present electrospraying method and apparatus, a number of gene specimens to be transferred into cells can be conveniently and rapidly transferred into cells with high transfer efficiency.

### BEST MODE FOR CARRYING OUT THE INVENTION

Hereinafter, the embodiment of the present invention will be described in detail. The present invention is a method for minimizing degradation of a gene which is a substance to be transferred into cells when electrospray is conducted, and an apparatus thereof. Examples of the gene to be transferred into cells according to the present invention include nucleobases such as DNA, RNA and analogs or derivatives thereof. These are provided in a form of plasmid, phage, virus, viroid, oligo DNA, oligo RNA, micro RNA or the like. Size of base sequence is not particularly limited. The base may be double-stranded or single-stranded. Also, it may be a nucleic acid analogue with a different main chain or a nucleic acid with an artificial base. Meanwhile, the method and apparatus of the present invention can be naturally used in order that others than genes, namely, protein, peptide, sugar, lipid, agricultural chemical, antimicrobial, metal ion, fluorescence-labeling reagent, isotope-labeling reagent or the like are transferred into cells.

The method and apparatus of the present invention are very effective when a gene is transferred into a cell by electrospraying a spray liquid free from the gene after the cell has been brought into contact with the gene. Meanwhile, this is also naturally effective when a spray liquid containing a gene to be transferred into a cell is electrosprayed on the cell.

By the way, the conventionally-used nozzle for electrospraying was a nozzle wholly made of an electrically conductive substance, a nozzle wholly made of an insulating substance which is provided with a lining of an electrically conductive material in the inside where the spray liquid passes, or a nozzle wholly made of an insulating substance which is provided with a thin tube made of an electrically conductive material inserted in the inside where the spray liquid passes, so as to apply a high voltage even to an ejection port region of the nozzle tip. Because of this, the electrically conductive part worked as a needle electrode to generate a corona discharge. However, the present inventor has now found out that an electric current resulting from the discharge causes the increase of molecular weight or decrease of molecular weight of the gene.

In addition, it has been found that a portion to which a high voltage is applied is not necessarily electrically connected with a portion for ejection, and if the two portions are devised to be positioned within a given distance, the spray liquid can be ejected by applying a high voltage whilst the discharge from the nozzle is avoided. That is, the method and apparatus of the present invention are the method and apparatus for transferring a gene into a cell, which comprises bringing a gene to be transferred into a cell in contact with the cell in a container, and then electrospraying the cell and gene in the container with a spray liquid free from the gene, characterized in that a nozzle is used which comprises a tube portion for applying a high voltage, which is made of an electrically conductive substance and located on the side of the spray liquid suction port, and a tube portion for spraying, which is made of an insulating substance and located on the side of the spray liquid ejection port.

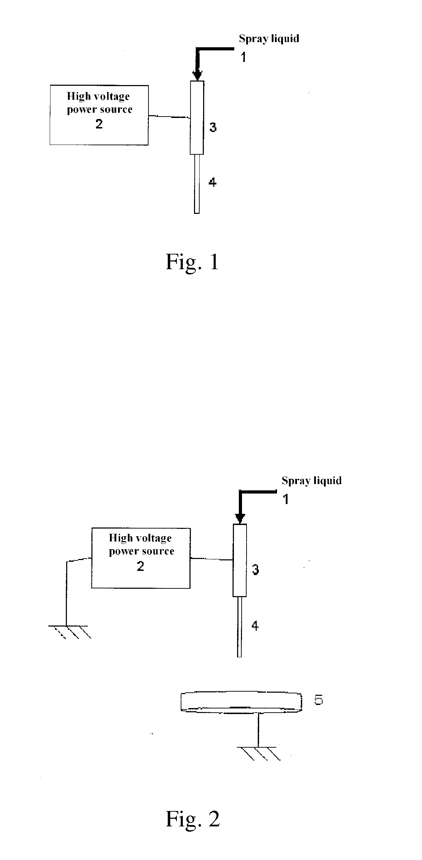

A concrete structure is shown in Fig. 1. It is a structure in which a spray liquid 1 becomes high in voltage at a high voltage application part 3 to which a high voltage is applied by a high voltage power source 2, and then electrosprayed from the tip of an insulating tube 4. Since the tube 4 is made of an insulating material, no electrical discharge occurs when there is no liquid inside, even if a high voltage is applied. Also, even when the inside of the tube 4 is filled with a spray liquid 1 at a high voltage, the insulating substance makes the amount of electric discharge smaller, compared with a case where the tube 4 is made of an electrically conductive material. Moreover, no electric shock may occur if one contacts the insulating tube 4 while a high voltage is applied.

A material for a tube portion which is made of an insulating substance and located on the side of the spray liquid ejection port is not particularly limited as long as it is an insulator. Preferable one is a plastic, and specifically includes phenol resin, melamine resin, urea resin, acryl resin, epoxy resin, polyolefin resin, styrene resin, vinylchloride resin, nylon resin, polycarbonate resin, polyacetal resin, fluorocarbon resin, polyether ketone resin or silicone resin. Particularly, polyethylene, polypropylene, Teflon (registered trade mark), polyether ketone or silicone is readily available and easy to use as a tube material.

By using the spraying nozzle comprising a tube which is made of an insulating substance on the side of the spray liquid ejection port, a discharge current from the nozzle tip can be decreased. An effective use condition of the present invention is shown in Fig. 2. It is a structure where the spray liquid 1 becomes high in voltage at the high voltage application part 3 to which a high voltage is applied by the high voltage power source 2, and is electrosprayed from the tip of the insulating tube 4 on the container 5 in which a cell and a gene as a substance to be transferred into the cell are placed. The container 5 is connected to ground potential so that no electric charge accumulates.

The inner diameter of the hole of the tip of the tube which is made of an insulating substance is preferably 1 µm to 10 mm and more preferably 0.1 mm to 5 mm. When the inner diameter of the hole is less than 1 µm, pressure loss during conveyance of the liquid may become large, whereas when it exceeds 10 mm, it may become difficult to spray the spray liquid, thereby requiring an excessively high speed of the liquid uneconomically. In addition, it is desirable to use a nozzle in which the shortest distance from the tube portion for applying a high voltage which is made of an electrically conductive substance and located on the side of the spray liquid suction port to the tube portion for spraying which is made of an insulating substance and located on the side of the spray liquid ejection port is 5 mm to 500 mm and preferably 10 mm to 200 mm. When it is shorter than 5 mm, risk of discharge is increased, whereas when it is longer than 500 mm, increase of pumping pressure applied to the spray liquid and inconvenience of operation may arise.

The voltage to be applied may be determined by finding an optimum value from the distances from cells, the tube size, and the flow rate, property or the like of the liquid to be electrosprayed, and is preferably 0.1 kV to 100 kV and more preferably 1 kV to 20 kV in terms of potential difference between the liquid of the tube tip and the cell. The polarity of the voltage applied to a tube may be either positive or negative.

The distance from the nozzle tip to the container in which cells are placed with the gene to be transferred into cells is preferably 1 to 200 mm and more preferably 5-100 mm. In contrast, in case a nozzle wholly made of an electrically conductive substance such as a metal nozzle is used and the distance between the two is adjusted to be not more than 200 mm, corona discharge starts immediately after the high voltage is applied, and corona discharge continues until the termination of the high voltage application. Thus, degradation of nucleobases becomes remarkable.

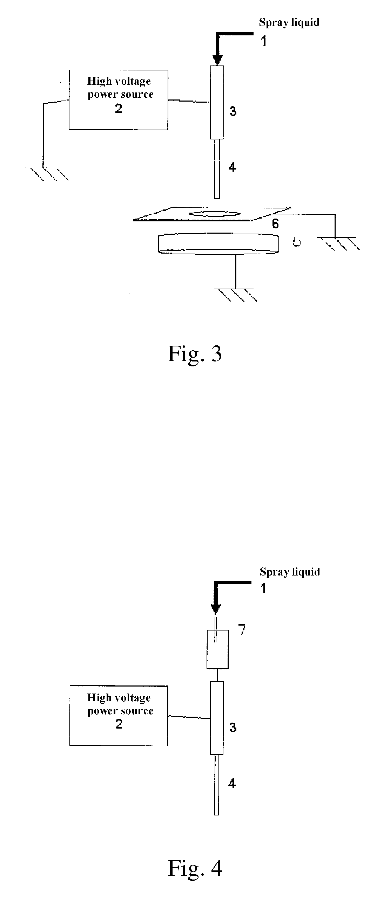

Further, as a method or means for controlling or inhibiting the degradation of the gene to be transferred into cells, it is effective to provide an electrode bypassing electric current between the spraying nozzle and the cells. An example thereof is shown in Fig. 3. It is a structure in which the spray liquid 1 becomes high in voltage at a high voltage application part 3 to which a high voltage is applied by a high voltage power source 2, and is electrosprayed from the tube tip 4 via the current-bypass electrode 6 on the container 5 in which the cell is placed. Meanwhile, the bypass electrode 6 is set to ground potential. The form thereof may be a structure through which a liquid to be electrosprayed is allowed to pass whereas electric current is bypassed, and for example, a perforated electrode made of an electrically conductive substance easily fulfills the role thereof. Also, a net electrode, a ring-shaped electrode or the like can fulfill the role thereof.

When the perforated electrode is used, diameter of perforation is preferably 3 mm to 300 mm and more preferably 5 mm 150 mm. When the diameter of perforations is less than 3 mm, the discharge current can be bypassed, but it becomes difficult to spray, decreasing the amount of the spray liquid sprayed on the cell. In contrast, when the diameter of perforations exceeds 300 mm, no obstacle to spraying arises, but it becomes difficult to catch discharge current for bypassing.

In this way, current flow in the container is inhibited and degradation of nucleobases is further decreased by using a bypass electrode in combination with the nozzle 4 made of an insulating substance as the structures that inhibit discharge current. Meanwhile, the position of the bypass electrode may be between the container in which a gene to be transferred into a cell is placed with the cell and the tip of the spray nozzle, and an optimum position may be selected according to the form thereof.

As the spray liquid used in the present invention, in most cases, an aqueous solution is used which contains an electrolyte to various extent and thus has electric conductivity. Thus, when a high voltage is applied to the solution, the whole liquid becomes high in voltage, and is accompanied by a risk of incurring electric shock or damage to electric circuit. As a countermeasure thereof, for example, an insulating part comprising a drip structure can be provided between the spray liquid transfer line and the tube portion for applying a high voltage which is made of an electrically conductive substance so as to cut off the conduction of the spray liquid, thereby preventing electric leakage. Therefore, a risk of electric shock and an amount of the spray liquid to be applied per unit of time can be decreased. It is concretely shown in Fig. 4. The drip structure 7 is provided upstream of the high voltage application part 3, so that the applied high voltage cannot be conducted through the spray liquid. A gas such as air and the like exists in the inside of the drip structure, and thus electrical insulation can be attained.

In order to inhibit degradation of nucleobases, it is important to make the electric current flowing through the cell and the gene to be transferred into the cell to be not more than 50 µA, and preferably to be not more than 20 µA desirably. As a method or means for controlling or inhibiting the let-through current other than the above bypass electrode, it is effective to provide a current output limit for the high voltage power source. In addition, the amount of integrated electric current is important. As the time for electrospraying on the cell and the gene to be transferred into the cell becomes longer, degradation becomes easier to proceed. In this sense, the electric current during electrospraying is preferably not more than 50 µA for an application time of preferably not more than 2 minutes, and more preferably not more than 20 µA for not more than 1 minute.

The present inventor assumes that degradation of nucleobases which occurs during electrospraying, that is, decrease and increase of molecular weight occur according to the following mechanism. That is, a tube to which a high voltage is applied causes corona discharge due to inhomogeneous electric field. Corona discharge generates radicals like plasma polymerization. The generated radicals cause hydrolysis, oxidative degradation or the like of DNA, so that molecular weight is decreased. Also, the generated radicals cause an intermolecular coupling reaction of nucleic acid portions, so that molecular weight is increased. In this way, it is assumed that, as a whole, plasma degradation and plasma polymerization progress at the same time. When degradation products of DNA produced under discharge conditions were subjected to electrophoresis at gel concentrations of 0.3% to 1% with an agarose gel of molecular weight measurement grade, no migration was observed in all cases. In addition, when the same DNA degradation products were treated with a restriction enzyme and loaded on the agarose gel, no migration was observed at all gel concentrations. From these results, it has been considered that denaturation into products that have not less than 60 kbp and are invulnerable to cleavage with restriction enzyme occurs due to degradation under discharge conditions.

The cell used in the present invention is not particularly limited, and can be any of the cells of animal, plant or microorganism. Also, it can be not only a cell but also tissue, organ or living body. Moreover, it is naturally possible to target a reproductive cell such as ovum, sperm, pollen, spore and seed.

Next, a method for transferring a gene into cells will be described in detail according to the operating procedures. Cells are exposed by removing the culture media. An aqueous solution containing a gene to be transferred into cells is placed therein so as to contact with the cells. Then, using an electrospray apparatus, the tube is moved so that the electrosprayed liquid is brought in contact with the whole cells. Thus, the transfer operation of the gene into cells is completed. After the completion, a culture medium is added to perform a culture of the cells into which the gene has been transferred. In this way, the gene can be safely and rapidly transferred into cells with high transfer efficiency while degradation is inhibited, by use of the electrospray method and apparatus of the present invention.

### EXAMPLE

Hereinafter, the present invention will be described in more detail by way of Examples and Comparative Examples. However, the present invention is in no way restricted to these Examples.

### Example 1

### Experiment of inhibition of DNA degradation

### (1) Experimental apparatus

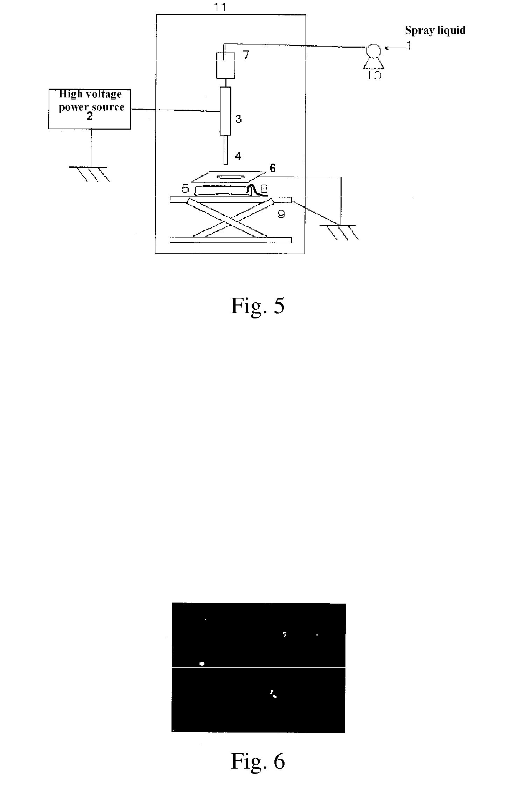

A structure of the electrospray apparatus used in the experiment is shown in Fig. 5. A spray liquid (water) 1 was introduced to a drip structure 7 via a tube pump 10. The dropped water was passed through the inside of a nickel-plated metal tube 3 which was located on the upper portion of the spraying nozzle. This metal tube was connected to a high voltage power source 2, and a high voltage was applied to water. Further, this metal tube was connected to a spraying tube 4 which was made of an insulating substance, and from which spraying was conducted via a perforated electrode 6 on a Petri dish 5 placed on a jack 9 which was set to ground potential. The inside of the petri dish was set to ground potential by way of a stainless steel ribbon 8. The whole apparatus was installed inside a clean booth 11. Meanwhile, in some experiments, the experiment was performed using the apparatus without the perforated electrode.

### (2) Experimental conditions

The experiment was performed without using the perforated electrode.

To a polystyrene petri dish with a diameter of 3.5 cm, 200 µL of water and 100 µL of 4.7 kbp plasmid DNA (10 µg/mL) were added. The supercoiled type structure constituted 9 µg, namely, 90% of 10 µg of the plasmid DNA used. 100 µL of water was sprayed at a flow rate of 100 µL/min from the height of 2 cm through a polypropylene tube with a length of 4 cm (outside diameter: 0.74 mm, inside diameter: 0.33 mm), at an applied voltage of -15kV. After spraying, 10 µL of a sample and 1 µL of a loading buffer were mixed, and 10 µL was electrophoresed. In this case, an electric current flowing through the petri dish due to the output of the high voltage power source was 28 µA. In this case, 7.2 µg of the supercoiled type remained. Degradation rate of the supercoiled type DNA was 20%.

### Comparative Example 1

The experiment was performed in the same manner as in Example 1. However, a stainless-steel tube with a length of 1.5 cm (outside diameter: 0.35 mm, inside diameter: 0.17 mm) was used for spraying. In this case, an electric current flowing through the petri dish due to the output of the high voltage power source was 57 µA. In this case, the amount of the remaining supercoiled type was 0.5 µg. Degradation of the supercoiled type DNA was 94%.

### Comparative Example 2

The experiment was performed in the same manner as in Example 1. However, water was not sprayed, and the apparatus was left for 5 minutes whilst the voltage was applied. A stainless-steel tube with a length of 1.5 cm (outside diameter: 0.35 mm, inside diameter: 0.17 mm) was used for spraying. In this instance, the flowed electric current was 61 µA. In this case, the amount of the remaining supercoiled type was 0.09 µg. Degradation rate of the supercoiled type DNA was 99%.

As a result of performing an electrophoresis using an agarose gel of 1% in concentration, a broad band corresponding to a range of about 500 to 5 kbp and a band which was not electrophoresed were observed. The broad band was in a region of a molecular weight lower than the plasmid, confirming that decrease of molecular weight occurred due to degradation.

Focusing on the band which was not electrophoresed, electrophoresis was conducted using an agarose gel of 0.3% instead of 1% in concentration so that a mass of 5 to 60 kbp could be analyzed in order to know how high molecular weight substances were yielded. However, it was not electrophoresed, and it was assumed that DNA was made high in molecular weight to have about more than 60 kbp by electric discharge. Therefore, it was shown from the results of electrophoresis that degradation of plasmid generates not only a DNA low in molecular weight but also degradation products high in molecular weight which do not migrate on electrophoresis.

### Example 2

A perforated electrode was used for the experiment.

The perforated electrode was a paper phenol copper clad laminate having a size of 100 mm x 100 mm, in which perforations of 4 cm in diameter were made. The height from the tube tip to a petri dish was set to 4 cm, in the middle of which this electrode was disposed and set to ground potential. 100 µL of a 30% TE solution of 4.7 kbp plasmid DNA (100 µg/mL) and 300 µL of water were placed in a polystyrene petri dish which was 3.5 cm in diameter and connected to a stainless steel ribbon leading to ground. 10 µg of the used plasmid DNA was constituted by 9.6 µg of the supercoiled ones.

100 µL of water was sprayed at a flow rate of 100 µL/min through a polypropylene tube with a length of 4 cm (outside diameter: 0.74 mm, inside diameter: 0.33 mm) at an applied voltage of -15 kV. In this instance, the electric current flowing through the petri dish was 4 µA. After spraying, the solution was collected and electrophoresed. As a result, an amount of the remaining supercoiled type was 9.6 µg, and thus no degradation occurred. Degradation rate was 0%.

### Example 3

The experiment was performed in the same manner as in Example 2. The perforated electrode was not used. In this instance, the current flowing through the petri dish was 19 µA. As a result, an amount of the remaining supercoiled type was 8.2 µg. Degradation rate was 15%.

### Comparative Example 3

An experiment was performed in the same manner as in Example 3. However, a stainless-steel tube with a length of 1.5 cm (outside diameter: 0.35 mm, inside diameter: 0.17 mm) was used for spraying. In this case, the electric current flowing through the petri dish was 25 µA. As a result, an amount of the remaining supercoiled type was 4.4 µg. Degradation rate was 54%.

### Example 4

### Experiment for a gene transfer

The same apparatus as Example 1 was used.

A plasmid (4.7 kpb) incorporated with a green fluorescence protein gene was used as a substance to be transferred into cells. Fibroblast (CHO cell) established from Chinese hamster ovary on biopsy was used as the cells. CHO cells were seeded on a polystyrene petri dish with a diameter of 3.5 cm and cultured in a medium composed of α-MEM medium + 10% FBS in a CO₂ incubator at 37°C. The medium for the cells was removed at a cell concentration of 100 x 10⁴, and 100 µL of the plasmid having a concentration of 100 µg/mL was added thereto.

100 µL of water was sprayed at a distance of 2 cm from the tube tip to the petri dish at a flow rate of 100 µL/min. At this time, -15 kV was applied to the tube. Water in the form of electrospray was sprayed from the tube on the petri dish. After spraying, the same medium was placed in the petri dish, and further culture was performed for one day. As a result of observation with a fluorescence microscope, transfer of the gene was confirmed by observation of cells generating a fluorescence due to a fluorescence protein, as shown in Fig. 6.

### BRIEF DESCRIPTION FOR DRAWINGS

Fig. 1 is a schematic view of an apparatus for electrospraying;
Fig. 2 is an apparatus for electrospraying, in which a petri dish is set to ground potential;
Fig. 3 is an apparatus for electrospraying with a bypass electrode;
Fig. 4 is an apparatus for electrospraying with a drip structure;
Fig. 5 is an apparatus for electrospraying used in the experiments; and
Fig. 6 is a fluorescence microscopic photograph (a photograph of cells expressing a fluorescent protein which was photographed using a microscope TE-2000S manufactured by NIKON with objective lens x 10, a fluorescence filter GFP block manufactured by NIKON, and a high voltage mercury lamp as a light source)

### DESCRIPTION OF SYMBOLS

1 denotes a spray liquid, 2 denotes a high voltage power source, 3 denotes a high voltage application part, 4 denotes a tube made of an insulating material, 5 denotes a petri dish, 6 denotes a bypass electrode, 7 denotes a drip structure, 8 denotes a stainless steel ribbon, 9 denotes a jack, 10 denotes a pump, and 11 denotes a clean booth.

## Claims

1. A method for transferring a gene into a cell by electrospraying, which comprises bringing a gene to be transferred into a cell in contact with the cell in a container, and then electrospraying the cell and gene in the container with a spray liquid free from the gene,
in which, as a nozzle for electrospraying, a nozzle is used which comprises:
a tube portion for applying a high voltage, which is made of an electrically conductive substance and located on the side of the spray liquid suction port, and
a tube portion for spraying, which is made of an insulating substance and located on the side of the spray liquid ejection port.

2. The method for transferring a gene into a cell by electrospraying, according to claim 1, wherein, the a nozzle for electrospraying, a nozzle is used in which the shortest distance from the tube portion for applying a high voltage, which is made of an electrically conductive substance and located on the side of the spray liquid suction port, to the tip of the ejection port of the tube portion for spraying, which is made of an insulating substance and located on the side of the spray liquid ejection port, is 5 mm to 500 mm.

3. The method for transferring a gene into a cell by electrospraying, according to claim 1, wherein electrospraying is conducted whilst an amount of electric current flowing through the cell and gene in the container is controlled not to exceed 50 µA.

4. The method for transferring a gene into a cell by electrospraying, according to claim 3, wherein an electrode through which electric current is bypassed is provided between the tip of the ejection port of the tube portion for spraying, which is made of an insulating substance and located on the side of the spray liquid ejection port, and the cell and gene placed in the container, and electrospraying is conducted whilst the amount of electric current flowing through the cell and gene is controlled not to exceed 50 µA.

5. The method for transferring a gene into a cell by electrospraying, according to claim 1, wherein a structure for dropping a spray liquid so as to cut off electrically conduction of the spray liquid is provided between the spray liquid suction port of the nozzle for electrospraying and a spray liquid transfer line arranged upstream of the suction port, whereby electrospraying is conducted with electric leak being prevented.

6. An apparatus which is used for conducting the method of transferring a gene into a cell by electrospraying according to any one of claims 1 to 5.

7. A nozzle for electrospraying, which comprises:
a tube portion for applying a high voltage, which is made of an electrically conductive substance and located on the side of the spray liquid suction port, and
a tube portion for spraying, which is made of an insulating substance and located on the side of the spray liquid ejection port.

8. The nozzle for electrospraying, according to claim 7, wherein the shortest distance from the tube portion for applying a high voltage to the tip of the ejection port of the tube portion for spraying is 5 mm to 500 mm.

9. An apparatus for transferring a gene into a cell, which comprises at least a container in which a cell and a gene to be transferred into the cell are placed, a nozzle for electrospraying the cell and gene in the container with a spray liquid free from the gene, and a high voltage power source which applies a high voltage to the nozzle, wherein the nozzle is a nozzle for electrospraying according to claims 7 and 8.

10. The apparatus according to claim 9, which further comprises a means which controls an amount of electric current flowing through the cell and gene in the container not to exceed 50 µA.

11. The apparatus according to claim 10, wherein the means which controls the amount of electric current not to exceed 50 µA comprises an electrode through which electric current is bypassed and which is provided between the tip of the ejection port of the tube portion for spraying and the container.

12. The apparatus according to any one of claim 9 to 11, which further comprises a structure for dropping a spray liquid so as to cut off electrical conduction of the spray liquid as an electric leak preventing means between the spray liquid suction port side of the nozzle and the spray liquid transfer line arranged upstream of the suction port.
